# EUROPEAN PATENT SPECIFICATION

(11) **EP 1 492 892 B1**
(45) Date of publication and mention of the grant of the patent: **05.08.2009**
(21) Application number: 03745687.8
(22) Date of filing: 04.02.2003
(51) Int. Cl.: C13K 1/00, C13K 1/08, C13K 3/00, C13K 7/00, C13K 11/00, C13K 13/00, C13D 1/00, C13D 3/00, C13D 3/14, C07H 1/06, C07H 3/00

(54) **PURIFICATION OF PURE DISACCHARIDE SOLUTION**
AUFREINIGUNG EINER REINEN DISACCHARIDLÖSUNG
PURIFICATION D'UNE SOLUTION DE DISACCHARIDE PURE

(30) Priority: 09.04.2002 FI 20020675
(43) Date of publication of application: 05.01.2005
(73) Proprietor: Danisco Sweeteners Oy, 48310 Kotka (FI)
(72) Inventor: RAVANKO, Vili, Clinton, IA 52732 (US); MÄYRÄ, Nina, FIN-00330 Helsinki (FI); HEIKKILÄ, Heikki, FIN-02320 Espoo (FI); KOIVIKKO, Hannu, FIN-02460 Kantvik (FI); KEKKI, Pekka, FIN-30100 Forssa (FI); KALLIOMÄKI, Hannu, FIN-30100 Forssa (FI); TYLLI, Matti, FIN-02460 Kantvik (FI); NYGREN, Johanna, FIN-08150 Lohja (FI)
(74) Representative: HOFFMANN EITLE
(86) International application number: PCT/EP2003/001091
(87) International publication number: WO 2003/085139

(56) References cited:
- EP-A- 0 098 352
- US-A- 3 817 787
- US-A- 3 864 406
- US-A- 4 325 742
- US-A- 4 487 198
- US-A- 4 816 445
- US-A- 5 462 864

## Description

The present invention pertains to a chromatographic process for separating saccharide monomers from saccharide dimers and/or for separating saccharide trimers from saccharide dimers.

Saccharides in the context of the present invention may either be sugars or sugar derived alcohols. Such saccharides, and in particular disaccharides such as maltose and maltitol, have recently attracted increased attention as advantageous sweetening agents for various food stuffs, as well as for other applications.

To date, a number of processes for producing saccharides, and in particular disaccharides, are known. For example, processes for producing maltose syrups, pure maltose as well as maltitol syrups and pure maltitol, are known. The common denominator of these processes is that they use starch as the starting material. Commonly, starch of various origins is liquefied and the thus obtained liquefied starch is treated with various enzymes so as to produce a maltose containing product.

Depending on the process employed, these maltose containing products vary significantly not only in their maltose content but also in view of the amount of maltotriose and glucose contained therein.

In order to arrive at a useful maltose containing product, the known processes proposed various purification procedures such as crystallisation or chromatographic separation. Membrane separation processes have also been proposed. The products obtained after these purifications may then be used as they are obtained, or subjected to further treatment. For example, maltose containing products may, after ion exchange, be hydrogenated so as to obtain maltitol containing products. Depending on the purity these products must also be purified further.

US 4,487,198, for example, discloses a process for purifying maltose syrup from a feed containing at least 70 wt%-DS maltose (wt%-DS as used in this description, and the claims means wt% on a dry solids basis) in addition to glucose and dextrin by way of a chromatographic separation. More precisely this process uses a strongly acidic cation exchange resin having sulphonyl groups of an alkaline metal or alkaline earth metal form to generate five fractions from the feed material.

The first fraction is rich in dextrin, the second fraction contains dextrin and maltose, the third fraction is rich in maltose, the fourth fraction contains maltose and glucose and the fifth fraction contains glucose. The mixed fractions, i.e. the second and fourth fractions are returned into the column in order to eventually obtain a high maltose syrup.

A further process for purifying maltose containing feed solutions by way of chromatography is disclosed in US 4,970,002. Processes leading to crystalline maltose are disclosed in US 5,112,407 and US 4,816,445.

US 5,462,864 discloses a process for producing high purity maltitol, comprising the steps of liquefying starch, saccharifying the liquefied starch and reducing the product mixture obtained in the saccharification step in order to obtain a maltitol containing product. This US patent also contemplates a chromatographic purification of both the maltose containing product as well as the maltitol containing product.

A similar process for producing maltitol is disclosed in US 4,846,139. US 4,846,139 discloses a process for the preparation of crystalline maltitol comprising successively: catalytic hydrogenation of a saccharified starch milk, a step of chromatographic fractionation of the hydrogenated syrup, crystallisation and separation of the maltitol crystals and recycling the mother liquor of the crystallisation into the fractionation step. US-A-3 817 787 discloses a method for separating monosaccharides from a starch conversion syrup and US-A-3 864 406 discloses a method of separating mannitol and sorbitol from solutions consisting of both polyols and water. While the aforementioned techniques may have their merits, there still remains a need for further improvement. In particular, there is a strong need to increase the maltose content especially in view of the separation of the maltose from glucose and maltotriose. In fact, there is a general need for improvement in the separation of disaccharides from mono- and trisaccharides.

This separation is rather difficult according to known methods: sugar dimers such as maltose, sugar monomers such as glucose and sugar trimers such as maltotriose have rather similar retention times in prior art separation columns. In fact, this is not only true for sugar monomers, dimers and trimers, but also for saccharide monomers (like sugar alcohols), dimers and trimers in general.

Because of the similar retention times in prior art chromatographic separations one had to compromise on the yield in order to obtain a good purity or visa versa. In some circumstances however, this compromise has lead to less than satisfying results, as high demands on purity entailed very low yields and thus rather uneconomic processes.

A good example for this is the production of crystalline maltitol. Maltotritol, i.e. the hydrogenation product of maltotriose, inhibits the crystallisation of maltitol even in small quantities. Accordingly, it is very difficult to obtain crystalline maltitol in satisfying quantities in the presence of Maltotritol. Maltotritol or its precursor the maltotriose must therefore be removed to the largest possible extent. Similar difficulties have been found when crystallizing sucrose in the presence of raffinose (sugar beet process) or in the presence of kestoses (cane sugar process). The same problem exists also in the cellulose and hemicellulose hydrolyzates, which contain saccharides with various chain lengths as degradation products. Therefor, the need to remove trimers and monomers from dimer solutions exists in general.

In light of the above, the present invention therefore aims at providing an improved process for separating saccharide dimers from monomers, and saccharide trimers from dimers so as to obtain saccharide monomers, dimers and/or trimers with high purity in an economical process.

The process set forth in the claims solves this object. The process is based on the recognition that, depending on the saccharides to be separated, the degree of crosslinking of the ion exchange resin must be adjusted. Ion Exchange resin is preferably a gel type cation exchange resin, and most preferably a gel type strong acid cation exchange resin.

The process is a column separation method, where the column filling material is ion exchange resin. The ion exchange resin can be chosen from a cation exchange resins (with styrene or acryl skeleton) as strong acid cation exchange resins or weak acid cation exchange resins.

The improvement achieved by the present invention resides, in particular, in the effective separation of disaccharides or saccharide dimers from saccharide monomers, and the separation of saccharide dimers from trimers. At the same time the process according to the present invention avoids unnecessary dilution of the product fractions and thereby achieves the aforementioned advantages without sacrificing the overall performance, i.e. purity and yield. Further advantages of the present invention will be apparent from the following detailed description.

The above object is achieved by a chromatographic process. This process may be designed as a sequential simulated moving bed process, a continuous simulated moving bed process, a batch chromatographic process or variants and combinations of these.

In this process a feed solution is subjected to a chromatographic separation with the aid of a crosslinked ion exchange resin, which is preferably a gel type cation exchange resin and most preferably a gel type strong acid cation exchange resin. If the composition of the feed solution is such that saccharide monomers should be separated from saccharide dimers, then the degree of crosslinking in the cation exchange resin should be high. If the composition of the feed is such that saccharides dimers should be separated from saccharides trimers, then the degree of crosslinking of the cation exchange resin should be low.

It should thereby be understood that a separation of saccharide monomers from saccharides dimers can lead to a saccharide monomer product fraction and a mixed fraction containing saccharide dimers. It can also lead to a mixed fraction and a saccharide dimer product fraction. Such a separation can also lead to two product fractions, namely a monosaccharide product fraction and a saccharide dimer product fraction. The same considerations apply mutatis mutandis to separating saccharide dimers and saccharides trimers. It is of course, also possible to treat a feed solution comprising saccharide monomers, dimers and trimers by separating the saccharide monomers from a mixed fraction in a first step and then separating the dimers from the mixed fraction in a second step. Likewise, it is also possible to purify such a monomer, dimer and trimer mixture by first of all separating a trimer fraction from a mixed monomer and dimer fraction, and to fractionate the mixed monomer and dimer fraction in a second step.

A resin with a high degree of crosslinking is preferably a resin that has a degree of crosslinking of 5 to 8 %. A resin with a low degree of crosslinking preferably has a degree of crosslinking of 2 to 4.5 %. The term degree of *crosslinking'* as used herein is defined in accordance with H.-G. Elias, Macromoleküle, Hüthig & Wepf Verlag Basel : Heidelberg and New York, 4th edition, 1981.

According to this definition the degree of crosslinking means the weight ratio of the crosslinkable monomers to the total monomers. It is conveniently expressed in percent.

It has surprisingly been found that the previously difficult separations of saccharide monomers from saccharide dimers, and saccharide dimers from saccharide trimers can be improved dramatically when choosing a crosslinked ion exchange resin with a particular degree of crosslinking, which is preferably a gel type cation exchange resin and most preferably a gel type strong acid cation exchange resin.

That is, the separation of monomers from dimers is made effectively when using a ion exchange resin with a degree of crosslinking of 5 to 8 %. At the same time, saccharide dimers are separated effectively from saccharide trimers with a ion exchange resin having a degree of crosslinking of 2 to 4.5 %. The present invention's separations remove trisaccharides by at least 75% and monomers by at least 65%. Moreorer, the yield of dimers is over 85 % .

It is thus a particular advantage of the process according to the present invention that the use of a particular crosslinked cation exchange resin for a particular starting material, i.e. a particular separation purpose, leads to very favourable separations. The present invention thereby overcomes the difficulties encountered in the prior art methods. This is true both for the separation of saccharides such as sugar monomers, dimers, and trimers, as well as saccharides such as sugar alcohol monomers, dimers and trimers.

The feed solution for the process according to the present invention may be any solution containing saccharide monomers, dimers and/or trimers. Usually the saccharide dimer will be the major component and preferably be present in an amount of 65 to 85 wt%-DS (i.e. weight-% on dry substance) in the feed solution. The amount of saccharide monomers and/or trimers contained in the feed solution is not particularly limited. However, it is preferred if they are present in an amount that is less than that of the saccharide dimer.

The method according to the present invention is particularly useful for separating feed solutions containing a large amount of saccharide dimer, such as 65 to 85 wt% - DS and only minor amounts of saccharide monomers and/or trimers. In the present method the amount of saccharide monomers and/or trimers is less than 10 wt%-DS, preferably the amount of saccharide monomers and/or trimers is 3 wt%-DS or less, more preferably 2 wt%-DS or less and in particular 1.5 wt%-DS or less.

The feed solution may contain various solvents such as ethanol and/or water. However, aqueous solutions are preferred.

Typical feeds for obtaining maltose rich fractions are feed solutions obtained as the result of starch hydrolysis. For the purpose of the present invention, it is not critical what kind of starch is subjected to hydrolysis and both amylase rich and amylopectin rich starches can be used. Common sources for such starches are potatoes, barley, corn, rice, sago, tapioca and other natural products well known in the art.

In order to produce a starch hydrolysis one typically in a first step liquefies or gelatinises a starch slurry. This may be done according to methods well known in the art. For example, such liquefaction can be achieved by using liquefying enzymes, acids or simply by heating the starch slurry to elevated temperatures.

The effect of the liquefaction step is the fragmentation of the starch molecule. The resulting fragments are then degraded further with the aid of enzymes in the subsequent saccharification step. The saccharification step, which may also be carried out according to methods well known in the art, leads to a mixture comprising maltose, glucose, maltotriose and other polydextrins.

The saccharification is also typically effected enzymatically. For this purpose, the use of β-amylases and 1.6 glycosidase such as isoamylase and pullulanase has proven successful. The saccharification is also well known in the art (Starch: Chemistry and Technology Academic Press, 1984).

One embodiment to utilize the invention is to produce starch hydrolyzate with enzymes in a way, that maltose content is high but the content of impurities like trimers (e.g. maltotriose) and monomers (e.g. glucose) are in the low level. The chromatographic separation method of the invention is used to remove the impurity, which exists in the higher concentration by choosing the resin with the relevant crosslinking degree.

Another embodiment to utilize the invention is to apply the separation method to the hydrolyzate solution of cellulose and hemicellulose; cellulose hydrolyzate comprising glucose, cellobiose and cellotriose and hemicellulose hydrolyzate comprising e.g. xylose, xylobiose and xylotriose or other hemicellulose based monomers, dimers and trimers.

The method of the invention can be applied also for the separation of sugar molasses. In case of the sugar beet molasses the saccharides are glucose, fructose as monomers, sucrose as dimer and raffinose as trimer. In case of cane sugar molasses monomers are glucose and fructose, dimer is sucrose and trimers are kestoses.

For the purpose of the present invention it was found that it is particularly advantageous if the liquefied starch is treated as follows.

In a first step the liquefied starch is saccharified with pullulanase (e.g. Optimax^{®} optimalt^{®}, dosage typically 1 1/ton - DS) and β-amylase (e.g. BBA^{®} dosage typically 1 1/t - DS). It is also particularly advantageous if subsequently some low temperature α - amalyse (e.g. BAN^{®} dosage typically 0.01 1/t-DS) is added and a final saccharification is achieved by way of the addition of maltogenic α - amylase (e.g. Maltogenase^{®} dosage typically 1.5 1/t-DS). The incubation times between enzyme additions are typically 0 hour to 20 hours depending on the speed of the added enzyme.

Effecting the saccharification by way of sequential addition of the enzymes allows to avoid maltose losses. The simultaneous addition of enzymes often leads to excess production of glucose and/or maltotriose. It is especially favourable to delay the addition of low temperature α - amylase as this enzymes exhibits random endo-activity. Prolonged action of this enzymes leads to the formation of uneven starch chain ends, and thus to an increased formation of maltotriose as the β - amylase action will be stopped more often to form uneven chain ends.

Proceeding in the above described way is particular advantageous as it yields a maltose syrup with a maltose content of approx. 88 wt%-DS or more. At the same time, the maltotriose content is 0.9 wt%-DS or less. However, it must also be noted that reaching very low maltotriose levels requires more enzyme or a longer process time. In practice, it may therefore be more advantageous to compromise the maltotriose concentration rather than to increase the costs, by using more enzymes or allowing for a prolonged hydrolysis time.

It should also be noted that depending on the type of starch and the degree of liquefaction of the raw material, it may be advantageous to add pullulanase β -amylase and maltogenic α-amylase sequentially rather than simultaneously.

Generally speaking, it is favourable for the process according to the present invention to use a feed solution for the chromatographic separation with a dry substance content of 25 to 70 wt%, especially 35 to 55 wt%. However, the process according to the present invention is not particularly limited in this respect.

In the present invention's method a feed solution with a disaccharide content of more than 70 wt%-DS is used and in particular from 75 wt% to 90 wt% - DS, or even better 75 wt% to 85 wt%-DS.

The feed solution as described above is then subjected to the chromatographic process according to the present invention. As mentioned before, the chromatographic separation according to the present invention uses a crosslinked ion exchange resin preferably gel type cation exchange resin. This crosslinked cation exchange resin may either be a strong or a weak acid cation exchange resin with styrene or acrylic skeleton.

Weakly acidic cation exchange resins may favourably be used e.g. for the separation of more hydrophobic saccharides.

Weakly acid cation exchange resins are particularly useful for feeds containing hydrophobic monosaccharides such as deoxy, methyl and anhydro sugars, as well as sugar alcohols from more hydrophilic sugars.

Most preferably, the weakly acidic cation exchange resin is used for separating saccharides such as hexoses, including ketohexoses, aldohexoses, pentoses such as ketopentoses aldopentoses, corresponding sugars and sugar alcohols as well as mixtures thereof, e.g. glucose, fructose, rhamnose anhydrosorbitol, sorbitol, erythritol, inositol, arabinose, xylose and xylitol. Sucrose, betaine and amino acid containing solutions can also be separated advantageously. The weakly acid cation exchange resin can also be used for separating anhydrosugars from corresponding sugars as well as anhydrox sugar alcohols from corresponding sugar alcohol.

Preferably, the weakly acid ionic exchange resin is a crosslinked acrylic resin with carboxylic functional groups for example Finex CA 16 GC (8% DVB). The resin may be in the H⁺, K⁺, Na⁺, Mg²⁺, or Ca²⁺ form. It may also be used in other forms.

For the most part however, one will use strong acid cation exchange resins for the separation of the saccharides. Strong acid cation exchange resins on the basis of sulphonated styrene divinyl benzene copolymers are particularly useful in the context of the present invention. Examples of such resin are Finex CS 8 GC (4% DVB, particle size 0.36 mm, manufactured by Finex Ltd., Finland) Purolite PCR 664 (6.5% DVB, particle size 0.4 mm, manufactured by Purolite Co., USA) and Amberlite C 3120 (6% DVB, particle size 0.35mm, manufactured by Rohm and Haas, USA).

These resins are advantageously used in their alkaline metal or earth alkaline metal form, whereby the alkaline metal form should be understood so as to include the NH₄⁺ form as well. Typically, resins in the Na⁺, Ca²⁺ or Mg²⁺ forms are preferred.

As far as the amount of sulphonyl groups in the strongly acid cation exchange resins is concerned, it should be noted that usually the sulphonation of these styrene rings is close to 100%. However, lesser degrees of sulphonation can also be used within the context of the present invention, provided that such resins still allow the above object to be achieved.

The aforementioned resins are packed in a column which is loaded with the feed solution. Typically, a feed solution containing 20 to 80wt%-DS is loaded onto the column in an amount of 5 to 20 vol.% based on the volume of the column.

The temperature at which the process according to the present invention is performed is not particularly limited. However, it has been found that elevated temperatures such as temperatures of 60°C or more lead to better results. Particularly good results are obtained at temperatures of 75°C or more.

As the feed solutions according to the present invention are preferably aqueous feed solutions, it is also generally favourable to work at temperatures below 100°C. The best temperature for performing the chromatographic separation according to the present invention thus falls within the range of 65 to 90°C. For maltose and maltitol containing syrups the preferred temperature is 80°C or higher.

As mentioned before, the process according to the present invention may lead to fractions rich in saccharide monomers, dimers and/or trimers. The product fractions and in particular the saccharide dimer product fractions are thereby of particularly high purity. That is such fractions usually contain 90 to 96 wt%-DS or more product, e.g. disaccharide. At the same time, the amount of impurities e.g. saccharide monomers and/or trimers in a disaccharide product fraction, is extremely small.

In as much as the present invention pertains to product fractions, it should be borne in mind that this means a single fraction or a mixture of 2 or more fractions rich in the respective product. It may also mean fractions from consecutive feeds as well as fractions derived from fractions obtained in the chromatographic separation or membrane separation process e.g. by way of concentration.

In the present invention resins different cross-linking can be used in columns, which are operated in parallel or in series. Part of the resin beds in parallel or serial columns can consist of resin with high or low crosslinking.

The following examples illustrate the invention. It should be borne in mind that the procedures, individual process steps, conditions and numerical values indicated in these examples are representative of the present invention.

### EXAMPLES

### Example 1 - Preparation of a low maltotriose feed solution by adding the enzymes simultaneously

A hot (temp > 65°C) liquefied starch solution from starch liquefying process having a DE of 7,8 and a dry solids content of 25 wt% was adjusted to a pH of 5,5 and cooled to 58°C. At this temperature four enzymes 1,5 1/t dry solids (DS) Maltogenase™ 4000 L , 1 l/t-DS Promozyme® 600 L , 1 1/t-DS beta-amylase Optimalt® BBA and 0,01 l/t-DS BAN 240L were added simulteneously = 0 h. The composition of the solution changed as follows:

| Time | Oligo | Maltotriose | Maltose | Glucose | Sum |
|---|---|---|---|---|---|
| h | wt%-DS | wt%-DS | wt%-DS | wt%-DS | wt%-DS |
| 2 | 24,71 | 7,50 | 61,49 | 1,44 | 95,14 |
| 18 | 12,74 | 3,68 | 77,79 | 4,39 | 98,61 |
| 26 | 10,19 | 2,65 | 80,46 | 5,08 | 98,38 |
| 48 | 7,56 | 1,64 | 83,42 | 5,91 | 98,52 |
| 68 | 5,81 | 0,92 | 85,33 | 6,34 | 98,39 |

This solution can be subjected to the chromatographic separation with high DVB-resin in order to remove glucose.

### Example 2 - Low maltotriose feed solution by delayed Addition of low temperature a-amylase

Example 1 was repeated. However, the temperature was set to 60°C and the dry matter content was increased from 25 to 30wt%. In addition, a different pullulanase, Optimax L-1000 (Genencor) was used and Novo BAN 240 L (low temperature - α-amylase) was added after 50 h from start of saccharification. The details and results are summarised in the table below.

| Enzyme | Time | Oligo | Maltotriose | Maltose | Glucose |
|---|---|---|---|---|---|
| 0 h : | h | wt%-DS | wt%-DS | wt%-DS | wt%-DS |
| | 2 | 12,0 | 7,2 | 72,2 | 1,4 |
| | 18 | 6,1 | 2,8 | 81,3 | 3,4 |
| | 24 | 5,8 | 2,0 | 82,8 | 3,8 |
| BAN 240 L | 50 | 4,8 | 0,9 | 87,5 | 4,7 |
| | 72 | 4,7 | 0,5 | 85,3 | 4,9 |
| | 144 | 4,9 | 0,1 | 85,8 | 5,4 |

This solution can be further treated similar than in Example 1 in order to obtain solution with high maltose purity.

### Example 3 - Low maltotriose feed solution by sequential addition of saccharifying enzymes

A liquefied barley starch solution having a DE of 4,6 was adjusted to 30wt%-DS and to pH 5,5 before enzyme addition. The temperature of the liquid was adjusted to 60°C and 1 1/t DS Optimax® L-1000 pullulanase (from Genencor) was added. After 23 hours at 60°C 1 1/t DS beta-amylase (Optimalt® BBA from Genencor) was added. After 30 hours 1,5 1/t DS maltogenic alpha-amylase (Maltogenase™ 4000 L from Novo) and 0,01 1/t DS low temperature alpha-amylase (BAN 240 L from Novo) were added. In total the product was incubated for 72 hours at 60°C. The composition developed as follows:

| | Maltotriose | Maltose | Glucose | >4 oligomers |
|---|---|---|---|---|
| h | wt%-DS | wt%-DS | wt%-DS | wt%-DS |
| 24 | 8,77 | 68,61 | 0,38 | 8,85 |
| 48 | 1,20 | 88,87 | 4,21 | 3,52 |
| 72 | 0,74 | 90,50 | 4,78 | 3,90 |

This solution is preferably further purified with chromatographic separation method with high DVB-resin to remove glucose.

### Example 4 - Low glucose high maltotriose feed solution

Liquefied corn starch having DE 10 was cooled to 60°C. The pH of the 25wt%-DS solution was adjusted to 5,5. Then 1 1/t DS Optimax® L-1000 pullulanase (from Genencor) was added. After 24 hours 1 1/t DS Optimally® BBA beta-amylase (from Genencor) was added. The hydrolysate was incubated for a total of 72 hours at 60°C. The composition developed as follows:

| | Maltotriose | Maltose | Glucose | >4 oligomers |
|---|---|---|---|---|
| h | wt%-DS | wt%-DS | wt%-DS | wt%-DS |
| 2 | 12,1 | 61,6 | 0,5 | 25,2 |
| 18 | 13,7 | 70,3 | 0,6 | 13,7 |
| 24 | 14,5 | 72,1 | 0,5 | 11,1 |
| 48 | 15,7 | 75,9 | 0,6 | 6,3 |
| 72 | 16,0 | 77,1 | 0,6 | 5,7 |

The solution is subjected to chromatographic separation with low DVB-resin to remove maltotriose.

### Example 5 - High maltose, low maltotriose feed solution by adding the enzymes sequentially at higher temperatures

Liquefied barley starch of 30wt%-DS with a DE of 2,6 was adjusted to pH 5,5 before enzyme additions. The temperature of the liquid was set to 65°C and a total of 0,4 l/t DS maltogenic alpha-amylase (Maltogenase™ 4000 L from Novo), 1 l/t DS beta-amylase (Optimalt® BBA from Genencor) and 1 l/t DS Optimax® L-1000 pullulanase (from Genencor) were added in four equal lots while stepwise lowering the temperature to 65, 64, 62, 60°C in 40 minutes intervals. After 12 hours 0,01 l/t DS low temperature alpha-amylase (BAN 240 L from Novo) was added. After 24 hours 1,1 l/t DS maltogenic alpha-amylase (Maltogenase® 4000 L from Novo) was added. In total the product was incubated for 100 hours. The composition developed as follows:

| | Maltotriose | Maltose | Glucose | >4 oligomers |
|---|---|---|---|---|
| h | wt%-DS | wt%-DS | wt%-DS | wt%-DS |
| 2 | 8,14 | 81,03 | 0,83 | 9,98 |
| 16 | 7,34 | 84,73 | 1,56 | 6,36 |
| 24 | 6,24 | 85,70 | 2,12 | 5,95 |
| 48 | 0,95 | 87,97 | 4,69 | 6,39 |
| 10 0 | 0,43 | 87,31 | 5,02 | 7,24 |

The solution can be subjected to chromatographic separation with high DVB-resin to remove glucose.

### Example 6 - Chromatographic separation of maltose solution with high glucose content

Starch hydrolysate (maltose hydrolysate) was subjected to a chromatographic separation in a batch separation column. The separation was performed in a pilot scale chromatographic separation column as a batch process.

The whole equipment consisted of a feed tank, a feed pump, a heat exchanger, a chromatographic separation column, product containers, pipelines for input of feed solution as well as eluent water, pipelines for output and flow control for the outcoming liquid.

The column with a diameter of 0,6 m was filled with a strong acid cation exchange resin. The height of the resin bed was approximately 5,2 m. The degree of cross-linkage was 5,5 w-% DVB and the average particle size of the resin was 0,35 mm. The resin was regenerated into sodium (Na⁺) form and a feeding device was placed at the top of the resin bed. The temperature of the column, feed solution and eluent water was 80°C. The flow rate in the column was adjusted to 210 l/h.

Chromatographic separation was carried out as follows:
- Step 1.: The dry substance of the feed solution was adjusted to 36 g dry substance in 100 g of solution according to the refractive index (RI) of the solution.
- Step 2.: 110 l of the preheated feed solution was pumped to the top of the resin bed.
- Step 3.: The feed solution was eluted downwards in the column by feeding preheated deionised water to the top of the column.
- Step 4.: The density and conductivity of the outcoming solution were measured continuously. The outcoming solution was collected and divided into five fractions in the following order: first residual fraction (containing oligosaccharides), recycle fraction (containing mostly maltose and maltotriose), maltose rich fraction (containing most of the maltose), second recycle fraction (containing mostly maltose and glucose) and second residual fraction (containing mostly glucose). Both recycle fractions were combined with the feed solution.

The amount of dry substance as well as maltose content in the feed solution and in product fraction are presented in the table below. The concentration of maltose is expressed as percentage of the total dry substance in the particular fraction. The yield of maltose in product fraction is also presented (the amount of the component in the particular fraction in relation to the total amount of that component in all product fractions excluding recycle fractions). Oligosaccharide, maltotriose and glucose removals are also presented. Removal is expressed as the amount of the component in residual fractions compared to the amount of that component in residual fractions and in product fraction.

| | Feed solution | Maltose fraction |
|---|---|---|
| DS in fraction, kg | 45 | 25 |
| DS g/100 g solution | 36 | 21 |
| Maltose wt%-DS | 82 | 97 |
| Maltotriose wt%-DS | 1,7 | 0,4 |
| Glucose wt%-DS | 6,1 | 1,9 |
| Oligosaccharides wt%-DS | 7,8 | 0,4 |
| Maltose yield % (with recycle) | | 97 |
| Oligosaccharide removal % | | 94 |
| Maltotriose removal % | | 69 |
| Glucose removal % | | 62 |

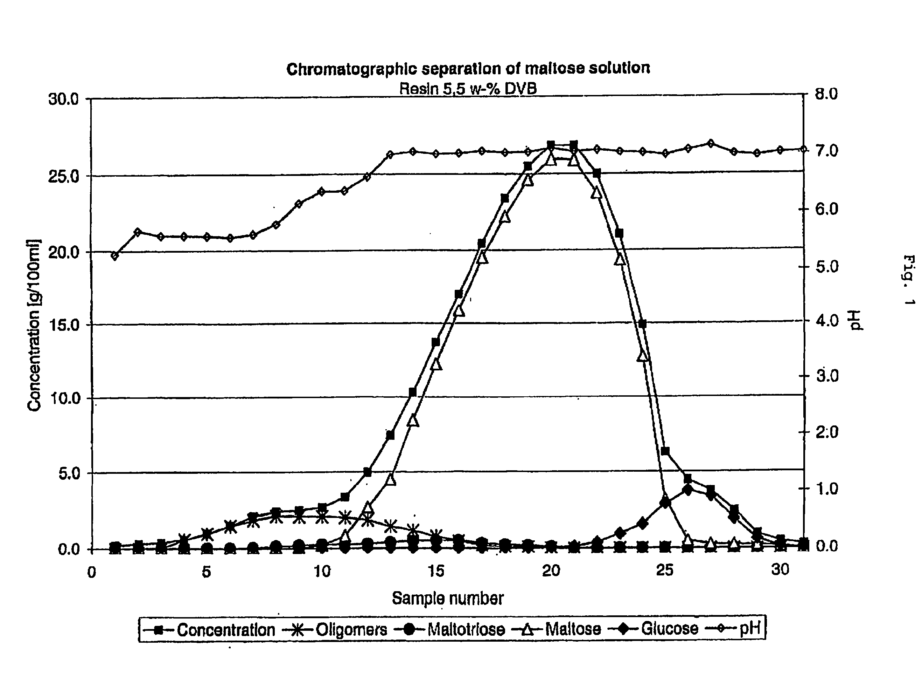

A resin with 5,5 w-% DVB separated well maltose from other components. Especially, the resin separated well maltose from glucose. Maltose purity was increased by 15%-units. Maltose yield was 97%. Results are shown in Fig. 1.

### Example 7 - Chromatographic separation of maltose solution with high glucose content

Starch hydrolysate (maltose hydrolysate) was subjected to a chromatographic separation in a batch separation column. The separation was performed in a pilot scale chromatographic separation column as a batch process.

The whole equipment consisted of a feed tank, a feed pump, a heat exchanger, a chromatographic separation column, product containers, pipelines for input of feed solution as well as eluent water, pipelines for output and flow control for the outcoming liquid.

The column with a diameter of 0,225 m was filled with a strong acid cation exchange resin. The height of the resin bed was approximately 5,2 m. The degree of cross-linkage was 4 w-% DVB and the average particle size of the resin was 0,36 mm. The resin was regenerated into sodium (Na⁺) form and a feeding device was placed at the top of the resin bed. The temperature of the column, feed solution and eluent water was 80°C. The flow rate in the column was adjusted to 30 l/h.

Chromatographic separation was carried out as follows:
- Step 1.: The dry substance of the feed solution was adjusted to 36 g dry substance in 100 g of solution according to the refractive index (RI) of the solution.
- Step 2.: 15 1 of the preheated feed solution was pumped to the top of the resin bed.
- Step 3.: The feed solution was eluted downwards in the column by feeding preheated ion-exchanged water to the top of the column.
- Step 4.: The density and conductivity of the outcoming solution were measured continuously. The outcoming solution was collected and divided into five fractions in the following order: first residual fraction (containing oligosaccharides), first recycle fraction (containing mostly maltose and maltotriose), maltose rich fraction (containing most of the maltose), second recycle fraction (containing mostly maltose and glucose) and second residual fraction (containing mostly glucose). First recycle fraction was introduced to the front slope and second recycle fraction to the back slope of the concentration profile of chromatographic separation.

Yields and composition of solution (maltose purity) are calculated similar than in Example 6.

| | Feed solution | Maltose fraction |
|---|---|---|
| DS in fraction, kg | 6,0 | 2,8 |
| DS g/100 g solution | 36 | 16 |
| Maltose wt%-DS | 83 | 89 |
| Maltotriose wt%-DS | 2,0 | 0,3 |
| Glucose wt%-DS | 6,5 | 9,0 |
| Oligosaccharides wt%-DS | 6,2 | 0,1 |
| Maltose, yield % | | 84 |
| Oligosaccharide removal % | | 99 |
| Maltotriose removal % | | 92 |
| Glucose removal % | | 6,1 |

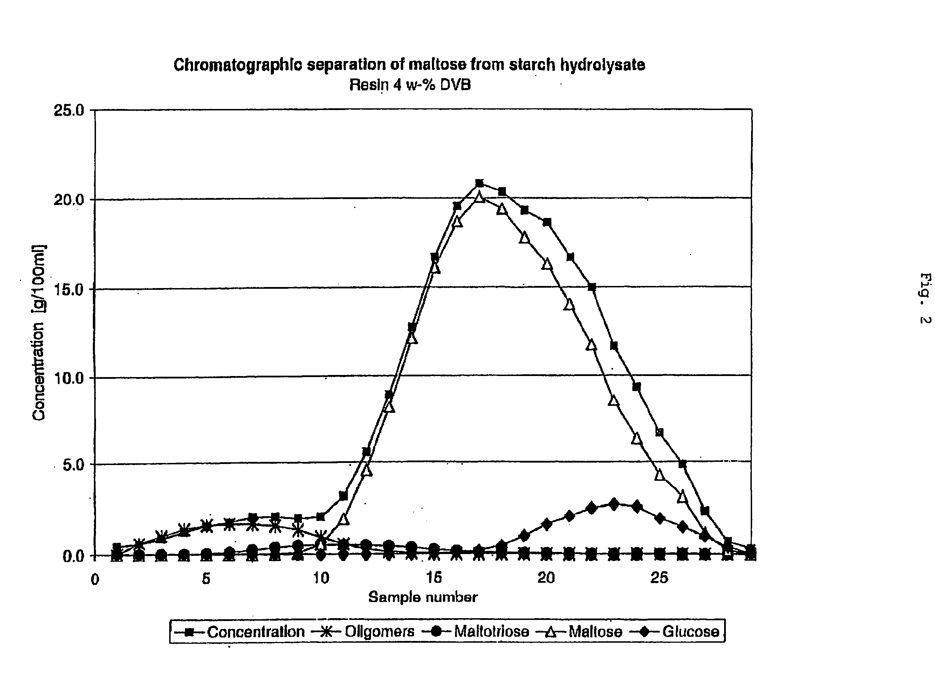

A resin with 4 w-% DVB separated well maltose from other components. Especially, the resin separated well maltose from oligosaccharides and maltoriose. Maltose purity was increased by 6%-units. Maltose yield was (84%). Results are shown in Fig. 2.

### Example 8 - Chromatographic separation of fructose run-off

Fructose run-off from fructose crystallization of a process based on sucrose, was subjected to a chromatographic separation in a batch separation column. The separation was performed in a pilot scale chromatographic separation column as a batch process.

The whole equipment consisted of a feed tank, a feed pump, a heat exchanger, a chromatographic separation column, product containers, pipelines for input of feed solution as well as eluent water, pipelines for output and flow control for the outcoming liquid.

The column with a diameter of 0,225 m was filled with a weakly acid cation exchange resin. The height of the resin bed was approximately 5,2 m. The degree of cross-linkage was 8 w-% DVB and the average particle size of the resin was 0,29 mm. The resin was regenerated into sodium (Na⁺) form and a feeding device was placed at the top of the resin bed. The temperature of the column, feed solution and eluent water was 65°C. The flow rate in the column was adjusted to 30 1/h. The pH of the resin was adjusted to approximately 4,5 by circulating acidic 5% Na-acetate solution through resin.

Chromatographic separation was carried out as follows:
- Step 1.: The dry substance of the feed solution was adjusted to 30 g dry substance in 100 g of solution according to the refractive index (RI) of the solution.
- Step 2.: 18 1 of the preheated feed solution was pumped to the top of the resin bed.
- Step 3.: The feed solution was eluted downwards in the column by feeding preheated deionised water to the top of the column.
- Step 4.: The density and conductivity of the outcoming solution were measured continuously. The outcoming solution was collected and divided into three fractions in the following order: residual fraction (containing mostly oligo- and disaccharides), recycle fraction (containing mostly glucose and fructose) and fructose rich fraction (containing most of the fructose). Recycle fraction was combined with the feed solution.

Yields and concentrations of components are calculated similar than in Example 6.

| | Feed solution | Fructose fraction |
|---|---|---|
| DS in fraction, kg | 6 | 4,2 |
| DS g/100 g solution | 30 | 10 |
| Fructose wt%-DS | 92 | 95 |
| Glucose wt%-DS | 2,2 | 1,6 |
| Oligo- and disaccharides wt%-DS | 2,2 | 0,2 |
| Fructose, yield % | | 96 |
| Oligo- and disaccharide removal % | | 97 |
| Glucose removal % | | 31 |

A resin with 8 w-% DVB separated well fructose from other components. Especially, the resin separated well fructose from oligo- and disaccharides. Fructose yield was 96%.

### Example 9 - High maltotriose and high glucose feed separations

Two different maltose hydrolysates were used. One contained more than 1,5wt%-DS maltotriose and the other contained more than 1,5wt%-glucose. The separation was carried out with a 4,0 DVB-% resin and a 5,5 DVB-% resin, respectively. The results are summarised in the table below.

| | | |
|---|---|---|
| Resin DVB-% | 4,0 | 5,5 |

| **Feed composition (wt%-DS)** | | |
|---|---|---|
| Maltose | 75,2 | 83,7 |
| Oligosaccharides | 7,3 | 7,2 |
| Maltotriose | 13,6 | 1,5 |
| Glucose | 1,2 | 6,3 |

| **Product composition (wt%-DS)** | | |
|---|---|---|
| Maltose | 91,0 | 91,0 |
| Oligosaccharides | 0,0 | 2,2 |
| Maltotriose | 3,1 | 1,2 |
| Glucose | 2,6 | 2,5 |
| | | |
| Maltose recovery (%) | 90 | 98,6 |
| Recycle ratio (%) | 18,2 | 0,0 |
| Oligosaccharide removal (%) | 100 | 72,5 |
| Maltotriose removal (%) | 70,4 | 24,8 |
| Glucose removal (%) | 6,5 | 60,4 |

### Example 10 - Purification and hydrogenation of maltose separation product

The maltose product from chromatographic separation process was purified using ion exchange as a tool. The resins in the purification step were strong acid cation exchange resin and weak base anion exchange resin. Te temperature during the purification was 60 degrees of centigrade and flow trough the resins was two bed volumes in hour. Feed syrup dry substance content was 50 %.

The hydrogenation was made in mixed batch autoclave at temperature of 115 degrees of centigrade and at 40 bar pressure using Raney nickel as a catalyst. The catalyst load was 10 % wet catalyst of batch dry substance. The hydrogenation time was four hours.

### Example 11 - Chromatographic separation of maltitol

Commercial maltitol syrup was subjected to a chromatographic separation in a batch separation column. The separation was performed in a pilot scale chromatographic separation column as a batch process.

The whole equipment consisted of a feed tank, a feed pump, a heat exchanger, a chromatographic separation column, product containers, pipelines for input of feed solution as well as eluent water, pipelines for output and flow control for the outcoming liquid.

The column with a diameter of 0,225 m was filled with a strong acid cation exchange resin. The height of the resin bed was approximately 5,2 m. The degree of cross-linkage was 4 w-% DVB and the average particle size of the resin was 0,36 mm. The resin was regenerated into sodium (Na⁺) form and a feeding device was placed at the top of the resin bed. The temperature of the column, feed solution and eluent water was 80°C. The flow rate in the column was adjusted to 30 1/h.

Chromatographic separation was carried out as follows:
- Step 1.: The dry substance of the feed solution was adjusted to 36 g dry substance in 100 g of solution according to the refractive index (RI) of the solution.
- Step 2.: 15 1 of the preheated feed solution was pumped to the top of the resin bed.
- Step 3.: The feed solution was eluted downwards in the column by feeding preheated deionised water to the top of the column.
- Step 4.: The density and conductivity of the outcoming solution were measured continuously. The outcoming solution was collected and divided into five fractions in the following order: first residual fraction (containing oligosaccharides), recycle fraction (containing mostly maltitol and maltotritol), maltitol rich fraction (containing most of the maltitol), second recycle fraction (containing mostly maltitol and sorbitol) and second residual fraction (containing mostly sorbitol). Both recycle fractions were combined with the feed solution.

Yields and concentrations of components are calculated equally than in Example 6.

| | Feed solution | Maltitol fraction |
|---|---|---|
| DS in fraction, kg | 6 | 2,7 |
| DS g/100 g solution | 36 | 16 |
| Maltitol wt%-DS | 63 | 94 |
| Maltotritol wt%-DS | 15 | 2,7 |
| Sorbitol wt%-DS | 3,9 | 0,8 |
| Oligosaccharides wt%-DS | 15 | 0,1 |
| Maltitol, yield % | | 89 |
| oligosaccharide removal % | | 100 |
| Maltotritol removal % | | 89 |
| Sorbitol removal % | | 88 |

A resin with 4 w-% DVB separated well maltitol from other components. Especially, the resin separated well maltitol from oligosaccharides and maltotritol. Maltitol purity was increased by 31%-units. Maltitol yield was 89%.

### Example 12 - Chromatographic separation of maltitol run-off, solution with low sorbitol content

Maltitol run-off from maltitol crystallization was subjected to a chromatographic separation in a batch separation column. The separation was performed in a pilot scale chromatographic separation column as a batch process.

The whole equipment consisted of a feed tank, a feed pump, a heat exchanger, a chromatographic separation column, product containers, pipelines for input of feed solution as well as eluent water, pipelines for output and flow control for the outcoming liquid.

The column with a diameter of 0,225 m was filled with a strong acid cation exchange resin. The height of the resin bed was approximately 5,2 m. The degree of cross-linkage was 4 w% DVB and the average particle size of the resin was 0,36 mm. The resin was regenerated into sodium (Na⁺) form and a feeding device was placed at the top of the resin bed. The temperature of the column, feed solution and eluent water was 80°C. The flow rate in the column was adjusted to 30 l/h.

Chromatographic separation was carried out as follows:
- Step 1.: The dry substance of the feed solution was adjusted to 36 g dry substance in 100 g of solution according to the refractive index (RI) of the solution.
- Step 2.: 9 liters of the preheated feed solution was pumped to the top of the resin bed.
- Step 3.: The feed solution was eluted downwards in the column by feeding preheated deionised water to the top of the column.
- Step 4.: The density and conductivity of the outcoming solution were measured continuously. The outcoming solution was collected and divided into five fractions in the following order: first residual fraction (containing oligosaccharides), recycle fraction (containing mostly maltitol and maltotritol), maltitol rich fraction (containing most of the maltitol), second recycle fraction (containing mostly maltitol and sorbitol) and second residual fraction (containing mostly sorbitol). Both recycle fractions were combined with the feed solution.

Yields and concentration of components have been calculated equally than in Example 6.

| | Feed solution | Maltitol fraction |
|---|---|---|
| DS in fraction, kg | 3,8 | 2,3 |
| DS g/100 g solution | 36 | 15 |
| Maltitol wt%-DS | 91 | 96 |
| Maltotritol wt%-DS | 6 | 0,6 |
| Sorbitol wt%-DS | 0,9 | 0,6 |
| Oligosaccharides wt%-DS | 0 | 0 |
| Maltitol, yield % | | 93 |
| Maltotritol removal % | | 91 |
| Sorbitol removal % | | 67 |

A resin with 4 w-% DVB separated well maltitol from other components. Especially, the resin separated well maltitol from maltotritol. Maltitol purity was increased by 5%-units. Maltitol yield was 93%.

### Example 13 - Maltitol crystallization

A crystallization test was made by using about 213 kg of maltitol feed syrup with purity 93,3 % and a dry substance concentration of about 63,2 wt.-%. The solution contained also sorbitol 3,0 wt%-DS, maltose 0,1wt%-DS and maltotriol 0,4wt%-DS.

The solution was continuously fed into a 400 liter evaporative vacuum crystallizer (boiling pan) where it was agitated and concentrated under reduced pressure by boiling. The liquid level was kept low by adjusting the feed rate. The seeding was made by 0,06% milled maltitol crystals at DS 78,2% at 60,2°C (supersaturation about 1,19). After seeding the crystal containing mass was further concentrated and agitated by boiling for 4,3 hours at about 60°C to DS 89,9% and the liquid level was increased at the same time. The temperature was controlled by pressure. Crystal size after boiling crystallization was 50-100µm.

After boiling the mass was divided into two cooling crystallizers. The main part of the mass was mixed at 60°C constant temperature overnight. Crystallization yield was 70 % M/M when calculated from mother liquor sample. Final crystal size after crystallization was 100-200µm.

Centrifugation was made 21 hours from the seeding with a laboratory centrifuge (basket Ø 23cm). The crystal cake assay without wash was 99,7 % DS and crystal yield was 66,9 % DS/DS.

The rest of the boiling crystallized mass was cooled from 60°C to 50°C linearly during 17 hours. The crystallization yield was 76,7 % M/M when calculated from mother liquor sample. The crystal size after cooling was 100-200µm.

Centrifuging was made 28 hours from seeding with the laboratory centrifuge (basket 0 23 cm). The crystal cake assay without wash was 97,4wt%-DS and the crystal yield was 73,5 % DS/DS. With 10% wash the assay of the cake was 98,6wt%-DS and yield 62,2wt% DS/DS.

## Claims

1. Chromatographic process for separating saccharide monomers from dimers and/or saccharide trimmers from dimers, in a feed solution having a disaccharide content of more than 70 wit. % DS, and an amount of saccharide monomers and/or trimers of less than 10 wt. % DS, wherein an ion exchange resin with a degree of crosslinking of 5 to 8 % is used when saccharide monomers are separated from dimers and an ion exchange resin with a degree of crosslinking of 2 to 4.5 % is used when saccharide trimers are separated from dimers, the process resulting in a separated disaccharide fraction by removal of at least 75 % of the saccharide trimers from the feed solution and/or by removal of at least 65 % of the saccharide monomers from the feed solution, and resulting in a dimer yield of over 85 %.

2. A process according to claim 1, wherein the feed solution has a disaccharide content of 75-90 wt. %.

3. A process according to claim 1 or claim 2, wherein the separated disaccharide fraction has a disaccharide content of 90-96 wt. % DS or more.

4. A process according to any preceding claim, wherein the feed solution has a saccharide monomer and/or saccharide trimer content of 3 wt. % DS or less.

5. A process according to claim 4, wherein the feed solution contains 2 wt. % DS or less of a saccharide monomer and/or saccharide trimer.

6. A process according to any one of the preceding claims, wherein the saccharide dimer is maltose, maltitol or sucrose.

7. A process according to any one of the preceding claims, wherein the saccharide dimer is cellobiose, cellobitol, xylobiose or xylobitol.

8. A process according to any one of the preceding claims, wherein the saccharide monomer is glucose, fructose or sorbitol.

9. A process according to any one of the preceding claims wherein the crosslinked cation exchange resin is a strong acid cation exchange resin.

10. A process according to claim 9, wherein the crosslinked cation exchange resin is a gel type strong acid cation exchange resin.

11. A process according to any one of the preceding claims, wherein the saccharides are derived from starch.

12. A process according to claim 11, wherein the saccharides are derived by saccharification of liquefied starch with pullulanase and beta-amylase.

13. A process according to claim 12, wherein the saccharides are derived further by treatment with maltogenic alpha-amylase and subsequent saccharification with low temperature alphy-amylase, optionally followed by a final saccharification with maltogenic alpha-amylase.

14. A process according to any one of the preceding claims, wherein the separation is effected at a temperature of 65 to 90 °C.

15. A process according to any one of the preceding claims, wherein the separation is effected at a temperature of 80 or more.

16. A process according to any one of the preceding claims, wherein the disaccharide is a sugar alcohol which process comprises the further step of crystallising the sugar alcohol.

17. A process according to claim 16, wherein the disaccharide sugar alcohol is maltitol.

## Patentansprüche

1. Chromatographisches Verfahren zum Abtrennen von Saccharidmonomeren von Dimeren und/oder Saccharidtrimeren von Dimeren in einer Ausgangslösung mit einem Disaccharidgehalt von mehr als 70 Gew.% DS und einer Menge an Saccharidmonomeren und/oder Trimeren von weniger als 10 Gew.% DS, wobei ein Ionenaustauschharz mit einem Vernetzungsgrad von 5 bis 8 % verwendet wird, wenn die Saccharidmonomere von den Dimeren abgetrennt werden, und ein Ionenaustauschharz mit einem Vernetzungsgrad von 2 bis 4,5 % verwendet wird, wenn die Saccharidtrimere von den Dimeren abgetrennt werden, wobei das Verfahren zu einer abgetrennten Disaccharidfraktion durch Entfernung von mindestens 75 % der Saccharidtrimere aus der Ausgangslösung und/oder durch Entfernung von mindestens 65 % der Saccharidmonomere aus der Ausgangslösung führt und zu einem Dimerertrag von mehr als 85 %.

2. Verfahren gemäß Anspruch 1, wobei die Ausgangslösung einen Disaccharidgehalt von 75 bis 90 Gew.% aufweist.

3. Verfahren gemäß Anspruch 1 oder 2, wobei die abgetrennte Disaccharidfraktion einen Disaccharidgehalt von 90 bis 96 Gew.% DS oder mehr aufweist.

4. Verfahren gemäß einem der vorstehenden Ansprüche, wobei die Ausgangslösung einen Saccharidmonomer- und/oder Saccharidtrimergehalt von 3 Gew.% DS oder weniger aufweist.

5. Verfahren gemäß Anspruch 4, wobei die Ausgangslösung 2 Gew.% DS oder weniger eines Saccharidmonomers und/oder Saccharidtrimers enthält.

6. Verfahren gemäß einem der vorstehenden Ansprüche, wobei das Sacchariddimer Maltose, Maltit oder Saccharose ist.

7. Verfahren gemäß einem der vorstehenden Ansprüche, wobei das Sacchariddimer Cellobiose; Cellobitol, Xylobiose oder Xylobitol ist.

8. Verfahren gemäß einem der vorstehenden Ansprüche, wobei das Saccharidmonomer Glucose, Fructose oder Sorbit ist.

9. Verfahren gemäß einem der vorstehenden Ansprüche, wobei das vernetzte Kationenaustauschharz ein stark saures Kationenaustauschharz ist.

10. Verfahren gemäß Anspruch 9, wobei das vernetzte Kationenaustauschharz ein stark saures Kationenaustauschharz vom Geltyp ist.

11. Verfahren gemäß einem der vorstehenden Ansprüche, wobei die Saccharide von Stärke abstammen.

12. Verfahren gemäß Anspruch 11, wobei die Saccharide von einer Saccharifizierung verflüssigter Stärke mit Pullulanase und beta-Amylase abstammen.

13. Verfahren gemäß Anspruch 12, wobei die Saccharide weiter durch Behandlung mit maltogener alpha-Amylase und darauffolgender Saccharifizierung mit Niedrigtemperaturalpha-Amylase abgeleitet sind, optional gefolgt von einer endgültigen Saccharifizierung mit maltogener alpha-Amylase.

14. Verfahren gemäß einem der vorstehenden Ansprüche, wobei die Abtrennung bei einer Temperatur von 65 bis 90°C bewirkt wird.

15. Verfahren gemäß einem der vorstehenden Ansprüche, wobei die Abtrennung bei einer Temperatur von 80°C oder mehr bewirkt wird.

16. Verfahren gemäß einem der vorstehenden Ansprüche, wobei das Disaccharid ein Zuckeralkohol ist, wobei das Verfahren den weiteren Schritt der Kristallisierung des Zuckeralkohols umfaßt.

17. Verfahren gemäß Anspruch 16, wobei der Disaccharidzuckeralkohol Maltit ist.

## Revendications

1. Procédé chromatographique pour séparer des monomères des dimères de saccharide et/ou des trimères des dimères de saccharide dans une solution d'alimentation qui présente une teneur en disaccharide de plus de 70 % en poids des solides secs et une quantité de monomères et/ou de trimères de saccharide de moins de 10 % en poids des solides secs,
dans lequel une résine échangeuse d'ions dont le degré de réticulation est compris entre 5 et 8 % est utilisée lorsque les monomères de saccharide sont séparés des dimères et une résine échangeuse d'ions dont le degré de réticulation est compris entre 2 et 4,5 % est utilisée lorsque les trimères de saccharide sont séparés des dimères,
le procédé fournissant une fraction de disaccharide séparée par l'enlèvement d'au moins 75 % des trimères de saccharide de la solution d'alimentation et/ou par l'enlèvement d'au moins 65 % des monomères de saccharide de la solution d'alimentation et qui permet d'obtenir un rendement en dimères de plus de 85 %.

2. Procédé selon la revendication 1, dans lequel la solution d'alimentation présente une teneur en disaccharide comprise entre 75 et 90 % en poids.

3. Procédé selon les revendications 1 ou 2, dans lequel la fraction de disaccharide séparée présente une teneur en disaccharide comprise entre 90 et 96 % en poids des solides secs ou plus.

4. Procédé selon l'une quelconque des revendications précédentes, dans lequel la solution d'alimentation présente une teneur en monomères de saccharide et/ou en trimères de saccharide d'au plus 3 % en poids des solides secs.

5. Procédé selon la revendication 4, dans lequel la solution d'alimentation contient au plus 2 % en poids des solides secs d'un monomère de saccharide et/ou d'un trimère de saccharide.

6. Procédé selon l'une quelconque des revendications précédentes, dans lequel le dimère de saccharide est le maltose, le maltitol ou le sucrose.

7. Procédé selon l'une quelconque des revendications précédentes, dans lequel le dimère de saccharide est la cellobiose, le cellobitol, le xylobiose ou le xylobitol.

8. Procédé selon l'une quelconque des revendications précédentes, dans lequel le monomère de saccharide est le glucose, le fructose ou le sorbitol.

9. Procédé selon l'une quelconque des revendications précédentes, dans lequel la résine réticulée échangeuse de cations est une résine d'échange de cations d'acide fort.

10. Procédé selon la revendication 9, dans lequel la résine réticulée échangeuse de cations est une résine de type gel échangeuse de cations d'acide fort.

11. Procédé selon l'une quelconque des revendications précédentes, dans lequel les saccharides dérivent de l'amidon.

12. Procédé selon la revendication 11, dans lequel les saccharides sont obtenus par saccharification de l'amidon liquéfié à l'aide de pullulanalse et de béta-amylase.

13. Procédé selon la revendication 12, dans lequel les saccharides sont obtenus en outre à l'aide d'un traitement à base d'alpha-amylases maltogènes et la saccharification qui suit est réalisée à l'aide d'alpha-amylases à basse température, ces étapes pouvant facultativement être suivies par une saccharification finale réalisée à l'aide d'une alpha-amylase maltogène.

14. Procédé selon l'une quelconque des revendications précédentes, dans lequel la séparation est effectuée à une température comprise entre 65° C et 90° C.

15. Procédé selon l'une quelconque des revendications précédentes, dans lequel la séparation est effectuée à une température de 80° C ou plus.

16. Procédé selon l'une quelconque des revendications précédentes, dans lequel le disaccharide est un alcool de sucre, lequel procédé comprend l'étape supplémentaire qui consiste à cristalliser l'alcool de sucre.

17. Procédé selon la revendication 16, dans lequel l'alcool de sucre disaccharide est le maltitol.
